# EUROPEAN PATENT APPLICATION

(11) **EP 4 059 919 A1**
(43) Date of publication of application: **21.09.2022**
(21) Application number: 21197194.0
(22) Date of filing: 16.09.2021
(51) Int. Cl.: C07C 51/09, C07C 51/41, C07C 63/26, C07C 63/28, C07F 5/00

(54) **POROUS MAGNETIC CARBON COMPOSITE, METHOD FOR PREPARING SAME, AND METHOD FOR PREPARING ALUMINUM-BASED METAL-ORGANIC FRAMEWORK WITH IRON OXIDE BONDED USING WASTE PET BOTTLE**

(30) Priority: 19.03.2021 KR 20210035910
(71) Applicant: Korea Institute of Science and Technology, Seoul 02792 (KR)
(72) Inventor: JUNG, Kyung-Won, 02792 Seoul (KR); CHO, Eun, 02792 Seoul (KR); CHOI, Jae Woo, 02792 Seoul (KR)
(74) Representative: advotec.

(57) **Abstract**

The present disclosure relates to a porous magnetic carbon composite, a method for preparing the same, and a method for preparing an aluminum-based metal-organic framework with iron oxide bonded using waste PET bottles, which allow cost reduction and simplification of metal-organic framework synthesis by conducting extraction of terephthalic acid from waste PET bottles and synthesis of a metal-organic framework using the terephthalic acid at the same time. The method for preparing an aluminum-based metal-organic framework with iron oxide bonded using waste PET bottles according to the present disclosure includes: a step of preparing an aqueous solution wherein a powder of waste PET bottles, an aluminum salt and an iron salt are mixed; and a step of forming an aluminum-based metal-organic framework with an iron oxide (αFe₂O₃) bonded by conducting hydrothermal synthesis at a temperature satisfying both the depolymerization temperature of PET and the synthesis temperature of an aluminum-based metal-organic framework in the aqueous solution, wherein, in the step wherein the aluminum-based metal-organic framework with an iron oxide (α-Fe₂O₃) bonded is formed by hydrothermal synthesis, the aluminum-based metal-organic framework is formed by reaction between terephthalic acid and aluminum as terephthalic acid is formed from the waste PET bottles, and an iron component of the iron salt is converted to α-Fe₂O₃ (hematite) and then bonded with the aluminum-based metal-organic framework.

## Description

### [Technical Field]

### [Explanation of National-supported Research and Development]

This study was supported by the Ministry of Science, ICT (Development of customized module technology to secure efficient field applicability of extreme environment responsive filter, Project No. 1711128476) under the superintendence of National Research Foundation of Korea.

The present disclosure relates to a porous magnetic carbon composite, a method for preparing the same, and a method for preparing an aluminum-based metal-organic framework with iron oxide bonded using waste PET bottles. More particularly, it relates to a porous magnetic carbon composite, a method for preparing the same, and a method for preparing an aluminum-based metal-organic framework with iron oxide bonded using waste PET bottles, which allow cost reduction and simplification of metal-organic framework synthesis by conducting extraction of terephthalic acid from waste PET bottles and synthesis of a metal-organic framework using the terephthalic acid at the same time.

### [Background Art]

A metal-organic framework (MOF) is a crystalline porous material wherein metal ions or metal oxides are linked to each other by organic ligands. Recently, the metal-organic framework is studied actively since it can be applied to adsorbents, gas storage materials, sensors, separation membranes, functional thin films, heterogeneous catalysts, conductors, etc. due to large internal surface area and various structural and chemical characteristics.

Terephthalic acid is the typical organic ligand of the metal-organic framework. However, there is a limitation in large-scale production of the metal-organic framework because the terephthalic acid is expensive.

The applicant has presented a method for extracting terephthalic acid through alkaline hydrolysis of waste PET bottles and synthesizing a metal-organic framework using the extracted terephthalic acid (Synthesis of magnetic porous carbon composite derived from metal-organic framework using recovered terephthalic acid from polyethylene terephthalate (PET) waste bottles as organic ligand and its potential as adsorbent for antibiotic tetracycline hydrochloride, Composites Part B: Engineering Volume 187, 15 April 2020, 107867, hereinafter referred to as non-patent document 1). This can remarkably reduce the production cost of a metal-organic framework because terephthalic acid is extracted from waste PET bottles.

The above method presented by the applicant requires a two-step process in synthesizing the metal-organic framework, i.e., extraction of terephthalic acid through alkaline hydrolysis of waste PET bottles and synthesis of a metal-organic framework using the extracted terephthalic acid.

### [References of Related Art]

### [Non-patent Documents]

(Non-patent document 1) Synthesis of magnetic porous carbon composite derived from metal-organic framework using recovered terephthalic acid from polyethylene terephthalate (PET) waste bottles as organic ligand and its potential as adsorbent for antibiotic tetracycline hydrochloride, Composites Part B: Engineering Volume 187, 15 April 2020, 107867.

### [Disclosure]

### [Technical Problem]

The present disclosure is directed to providing a method for preparing an aluminum-based metal-organic framework with iron oxide bonded using waste PET bottles, which allows cost reduction and simplification of metal-organic framework synthesis by conducting extraction of terephthalic acid from waste PET bottles and synthesis of a metal-organic framework using the terephthalic acid at the same time.

The present disclosure is also directed to providing a porous magnetic carbon composite having superior adsorption performance for organic pollutants and heavy metals in water by sintering an aluminum-based metal-organic framework with iron oxide bonded using waste PET bottles under an optimized condition, and a method for preparing the same.

### [Technical Solution]

A method for preparing an aluminum-based metal-organic framework with iron oxide bonded using waste PET bottles according to the present disclosure includes: a step of preparing an aqueous solution wherein a powder of waste PET bottles, an aluminum salt and an iron salt are mixed; and a step of forming an aluminum-based metal-organic framework with an iron oxide (α-Fe₂O₃) bonded by conducting hydrothermal synthesis at a temperature satisfying both the depolymerization temperature of PET and the synthesis temperature of an aluminum-based metal-organic framework in the aqueous solution, wherein, in the step wherein the aluminum-based metal-organic framework with an iron oxide (α-Fe₂O₃) bonded is formed by hydrothermal synthesis, the aluminum-based metal-organic framework is formed by reaction between terephthalic acid and aluminum as terephthalic acid is formed from the waste PET bottles, and an iron component of the iron salt is converted to α-Fe₂O₃ (hematite) and then bonded with the aluminum-based metal-organic framework.

The depolymerization temperature range of PET overlaps with the temperature range where the aluminum-based metal-organic framework is synthesized, and the hydrothermal synthesis is conducted within the overlapping temperature range. The temperature range where the depolymerization temperature range of PET overlaps with the temperature range where the aluminum-based metal-organic framework is synthesized is 200-240 °C.

The aluminum salt and the iron salt contain a nitrate group (NO₃⁻). The aluminum salt may be Al(NO₃)₃·9H₂O and the iron salt may be Fe(NO₃)₃·9H₂O.

The method may include a step of, in the state where the aluminum-based metal-organic framework has been formed by hydrothermal synthesis, removing unreacted terephthalic acid remaining in the aluminum-based metal-organic framework and inducing activation of the aluminum-based metal-organic framework at the same time by activating the aluminum-based metal-organic framework at a predetermined temperature.

The activation temperature of the aluminum-based metal-organic framework is 300-350 °C.

In the aqueous solution wherein the powder of waste PET bottles, the aluminum salt and the iron salt are mixed, they are mixed at a molar ratio Al : Fe : TPA (terephthalic acid): H₂O = 1 : 1 : 0.3-0.7 : 48-112, and the addition amount of the powder of waste PET bottles is determined based on the molar ratio.

A method for preparing a porous magnetic carbon composite according to the present disclosure includes: a step of preparing an aqueous solution wherein a powder of waste PET bottles, an aluminum salt and an iron salt are mixed; a step of forming an aluminum-based metal-organic framework with an iron oxide (α-Fe₂O₃) bonded by conducting hydrothermal synthesis at a temperature satisfying both the depolymerization temperature of PET and the synthesis temperature of an aluminum-based metal-organic framework in the aqueous solution; and a step of forming a porous magnetic carbon composite with alumina and magnetite bonded by sintering the aluminum-based metal-organic framework with iron oxide bonded.

In the step wherein the aluminum-based metal-organic framework with an iron oxide (α-Fe₂O₃) bonded is formed by hydrothermal synthesis, the aluminum-based metal-organic framework is formed by reaction between terephthalic acid and aluminum as terephthalic acid is formed from the waste PET bottles, and an iron component of the iron salt is converted to α-Fe₂O₃ (hematite) and then bonded with the aluminum-based metal-organic framework.

As a result of the sintering of the aluminum-based metal-organic framework with iron oxide bonded, an organic ligand compound is converted to a carbon-based material, an aluminum compound of the metal-organic framework is converted to alumina (Al₂O₃) and the iron oxide (α-Fe₂O₃) bonded to the metal-organic framework is converted to magnetite (Fe₃O₄) with the porous structure of the metal-organic framework maintained.

The sintering temperature of the aluminum-based metal-organic framework with iron oxide bonded is 600-800 °C.

A porous magnetic carbon composite according to the present disclosure is a porous magnetic carbon composite with alumina and magnetite bonded formed by sintering of an aluminum-based metal-organic framework with iron oxide bonded, wherein the aluminum-based metal-organic framework with iron oxide bonded is formed by hydrothermal synthesis of an aqueous solution wherein a powder of waste PET bottles, an aluminum salt and an iron salt are mixed.

### [Advantageous Effects]

A porous magnetic carbon composite, a method for preparing the same and a method for preparing an aluminum-based metal-organic framework with iron oxide bonded using waste PET bottles according to the present disclosure provide the following effects.

The cost of preparing a metal-organic framework can be reduced and the method for preparing the metal-organic framework can be simplified since extraction of terephthalic acid from waste PET bottles and synthesis of the metal-organic framework using the terephthalic acid are conducted at the same time.

Since the porous magnetic carbon composite has large specific surface area because it is derived from a metal-organic framework, it has superior adsorption characteristics for organic pollutants and heavy metals in water owing to the chemical adsorption characteristics owing to the formation of graphite and metal oxides.

### [Brief Description of Drawings]

FIG. 1 shows a flow chart for describing a method for preparing an aluminum-based metal-organic framework with iron oxide bonded using waste PET bottles and a method for preparing a porous magnetic carbon composite according to an exemplary embodiment of the present disclosure.
FIG. 2 shows an XRD analysis result for iron oxide produced at the temperature where an aluminum-based metal-organic framework is synthesized.
FIG. 3 shows an XRD analysis result of an alumina-based metal-organic framework with iron oxide bonded prepared in Experimental Example 1.
FIG. 4 shows an XRD analysis result showing the physical property change of an alumina-based metal-organic framework with iron oxide bonded depending on activation temperature.
FIG. 5 shows a thermogravimetric analysis result of an alumina-based metal-organic framework depending on activation temperature.
FIG. 6 shows an XRD analysis result of a porous magnetic carbon composite depending on sintering temperature.
FIG. 7 shows the adsorption performance of a porous magnetic carbon composite according to Experimental Example 4.
FIG. 8A and FIG. 8B show a result of assessing the porosity of a porous magnetic carbon composite according to Experimental Example 5.
FIG. 9 shows a result of assessing the magnetism recovery of porous magnetic carbon composite according to Experimental Example 6.

### [Best Mode]

The present disclosure presents a technology about a porous magnetic carbon composite having superior adsorption performance for organic pollutants and heavy metals in water. The porous magnetic carbon composite according to the present disclosure has large specific surface area derived from a metal-organic framework and also has chemical adsorption characteristics resulting from sintering of the metal-organic framework.

As mentioned above in the 'Background Art' section, the applicant has presented a technology of extracting terephthalic acid, which is an organic ligand of a metal-organic framework (MOF), from waste PET bottles (see non-patent document 1). The present disclosure has improved the technology disclosed in the non-patent document 1 and presents a method for synthesizing a metal-organic framework from waste PET bottles via a single reaction by conducting extraction of terephthalic acid from waste PET bottle and synthesis of a metal-organic framework using the terephthalic acid at the same time.

The synthesis of the metal-organic framework from the waste PET bottles is possible via only a single reaction because the synthesis temperature of the aluminum (Al)-based metal-organic framework is very similar to the depolymerization of PET (polyethylene terephthalate).

PET is degraded into terephthalic acid when reacting with ethylene glycol. The depolymerization temperature of PET is about 220 °C. The synthesis temperature of an aluminum-based metal-organic framework wherein aluminum is used as a metal of the metal-organic framework is also known to be about 220 °C. Since the depolymerization temperature of PET is very similar to the synthesis temperature of the aluminum-based metal-organic framework, extraction of terephthalic acid through depolymerization of PET and synthesis of an aluminum-based metal-organic framework can be conducted at the same time in a specific temperature range. Therefore, according to the present disclosure, the two-step process as in the non-patent document 1, i.e., extraction of terephthalic acid from waste PET bottles through alkaline hydrolysis and synthesis of a metal-organic framework using the extracted terephthalic acid, is unnecessary.

In the present disclosure, the aluminum-based metal-organic framework has iron oxide and the iron oxide is converted to magnetite during sintering of the metal-organic framework. As described above, a porous magnetic carbon composite formed from the sintering of the aluminum-based metal-organic framework is used for adsorption of organic pollutants and heavy metals in water. The binding of magnetite exhibiting magnetism to the porous magnetic carbon composite allows easy recovery after the adsorption.

Hereinafter, a porous magnetic carbon composite, a method for preparing the same and a method for preparing an aluminum-based metal-organic framework with iron oxide bonded using waste PET bottles according to an exemplary embodiment of the present disclosure will be described in detail.

A method for preparing an aluminum-based metal-organic framework with iron oxide bonded using waste PET bottles and a method for preparing a porous magnetic carbon composite according to an exemplary embodiment of the present disclosure are conducted sequentially in time.

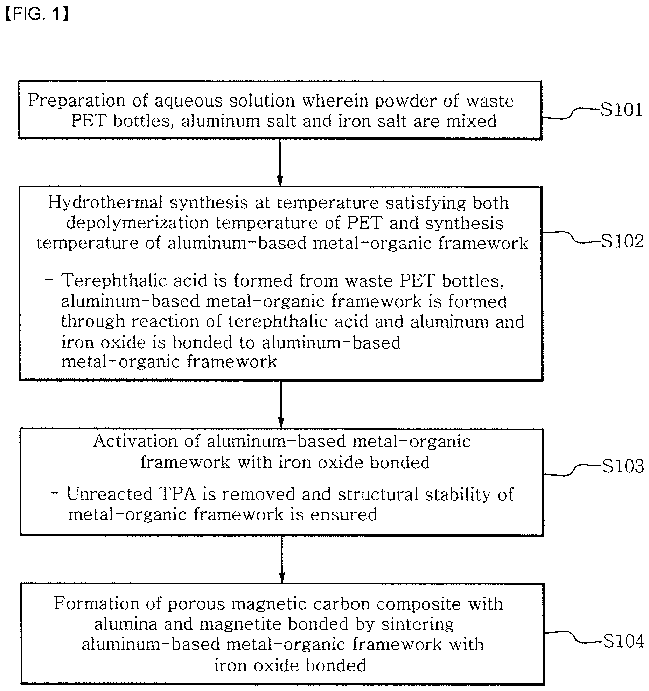

Referring to FIG. 1, a powder of waste PET bottles, an aluminum salt and an iron salt are prepared separately. Then, after dissolving the aluminum salt and the iron salt in water, the powder of waste PET bottles is added to the aqueous solution wherein the aluminum salt and the iron salt are dissolved (S101). After the powder of waste PET bottles has been added to the aqueous solution wherein the aluminum salt and the iron salt are dissolved, the aqueous solution is stirred for a predetermined time.

The powder of waste PET bottles may be prepared by crushing collected waste PET bottles using a crusher and screening a powder with a size of 1 mm or smaller through a sieve.

Specifically, the aluminum salt and the iron salt may be salts containing a nitrate group (NO3⁻). Al(NO₃)₃·9H₂O may be used as the aluminum salt and Fe(NO₃)₃·9H₂O may be used as the iron salt. The reason why the salts containing a nitrate group (NO₃⁻) are used as the aluminum salt and the iron salt is because NO₃⁻ facilitates the degradation of plastics during depolymerization of PET. That is to say, the depolymerization of PET is facilitated by NO₃⁻.

When mixing the aluminum salt, the iron salt and the powder of waste PET bottles, the mixing molar ratio is set to Al: Fe : TPA (terephthalic acid) : H₂O = 1 : 1 : 0.3-0.7 : 48-112. The addition amount of the powder of waste PET bottles is determined based on the molar ratio.

In the state where the aqueous solution wherein the aluminum salt and the iron salt are dissolved and the powder of waste PET bottles is mixed has been prepared, the aqueous solution is heat-treated at a predetermined temperature under a high pressure (S102). The heat treatment temperature corresponds to the depolymerization temperature of PET and the synthesis temperature of the aluminum-based metal-organic framework. The depolymerization temperature of PET and the synthesis temperature of the aluminum-based metal-organic framework are about 200-240 °C, respectively. The heat treatment of the aqueous solution under a high pressure may be performed in an autoclave.

Through the heat treatment of the aqueous solution under a high pressure, the depolymerization of PET and the synthesis of the aluminum-based metal-organic framework occur at the same time. That is to say, terephthalic acid is produced from the depolymerization of PET and the aluminum-based metal-organic framework is formed at the same time from the reaction of the terephthalic acid with aluminum. During this process, the nitrate contained in the aluminum salt and the iron salt facilitates the depolymerization of PET.

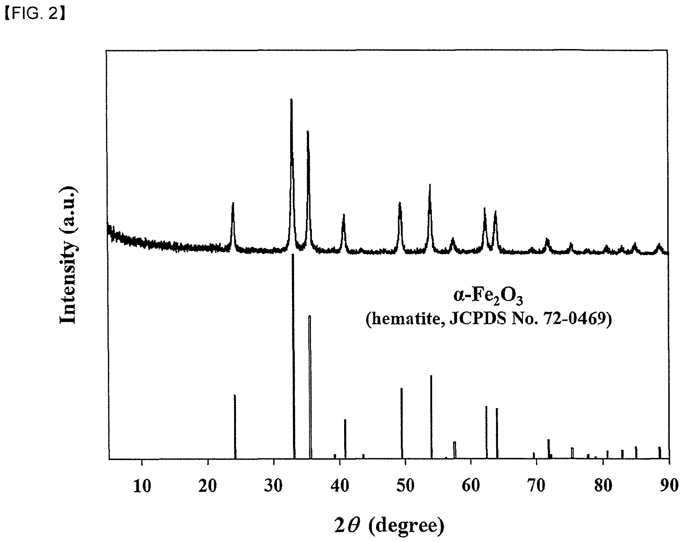

Iron is also contained in the aqueous solution in addition to aluminum. Through the heat treatment under a high pressure described above, iron (Fe) is converted to α-Fe₂O₃ (hematite), and the α-Fe₂O₃ is bonded with the aluminum-based metal-organic framework. Since the iron (Fe) is converted to α-Fe₂O₃ (hematite), an iron-based metal-organic framework is not formed (see FIG. 2).

That is to say, through the heat treatment of the aqueous solution under a high pressure, the depolymerization of PET and the synthesis of the aluminum-based metal-organic framework proceed at the same time and, as a result, the aluminum-based metal-organic framework is formed as the iron component of the iron salt is converted to α-Fe₂O₃ (hematite) and bonded with the aluminum-based metal-organic framework. Finally, an aluminum-based metal-organic framework with an iron oxide (α-Fe₂O₃) bonded is produced.

After the aluminum-based metal-organic framework with an iron oxide (α-Fe₂O₃) bonded has been produced, the aluminum-based metal-organic framework with an iron oxide (α-Fe₂O₃) bonded is separated by centrifugation, etc., washed using deionized water and ethanol, and then dried.

In this state, the aluminum-based metal-organic framework with an iron oxide (α-Fe₂O₃) bonded is activated (S103). The activation is conducted to remove unreacted terephthalic acid and induce the activation of the aluminum-based metal-organic framework with an iron oxide (α-Fe₂O₃) bonded.

Since the unreacted terephthalic acid blocks the pores of the aluminum-based metal-organic framework with an iron oxide (α-Fe₂O₃) bonded, it hinders the porous structure of the aluminum-based metal-organic framework which will be described later and inhibits the adsorption performance of the porous magnetic carbon composite prepared through a subsequent process. Accordingly, by conducting activation at 300-350 °C, the unreacted terephthalic acid is removed and the activation of the aluminum-based metal-organic framework with an iron oxide (α-Fe₂O₃) bonded is induced.

The method for preparing an aluminum-based metal-organic framework with iron oxide bonded using waste PET bottles according to an exemplary embodiment of the present disclosure is completed by the activation process.

In the state where the aluminum-based metal-organic framework with iron oxide bonded has been prepared using the waste PET bottles, a porous magnetic carbon composite is produced by sintering the prepared aluminum-based metal-organic framework with iron oxide bonded at a predetermined temperature under nitrogen atmosphere (S104).

Specifically, as a result of the sintering, the organic ligand compound is converted to a carbon compound and the aluminum compound of the metal-organic framework is converted to alumina (Al₂O₃) with the porous structure of the metal-organic framework maintained. In addition, the iron oxide (α-Fe₂O₃) bonded to the metal-organic framework is converted to magnetite (Fe₃O₄) exhibiting magnetism.

As described, the aluminum-based metal-organic framework with iron oxide bonded is converted to a porous magnetic carbon composite through the sintering, and alumina (Al₂O₃) and magnetite (Fe₃O₄) are bonded to the porous magnetic carbon composite with the porous structure of the metal-organic framework maintained.

The porous magnetic carbon composite formed from the sintering of the aluminum-based metal-organic framework with iron oxide bonded has large specific surface area derived from the metal-organic framework. As terephthalic acid is carbonized to graphite, etc., metal oxides such as alumina (Al₂O₃) and magnetite (Fe₃O₄) are formed and, as a result, various functional groups such as a hydroxy group (-OH), a benzene group, etc. are formed on the surface of the porous magnetic carbon composite. Accordingly, the porous magnetic carbon composite has chemical adsorption characteristics resulting from the formation of graphite and metal oxides in addition to the physical adsorption characteristics of the porous structure. Due to the physical adsorption characteristics and chemical adsorption characteristics, the porous magnetic carbon composite with alumina (Al₂O₃) and magnetite (Fe₃O₄) bonded according to the present disclosure exhibits very superior adsorption characteristics for organic pollutants and heavy metals in water. In particular, it exhibits superior adsorption performance for nonsteroidal anti-inflammatory drugs (NSAIDs) and hexavalent chromium (Cr(VI)). The adsorption performance of the porous magnetic carbon composite according to the present disclosure will be described in detail through experimental results.

The method for preparing an aluminum-based metal-organic framework with iron oxide bonded using waste PET bottles and the method for preparing a porous magnetic carbon composite according to an exemplary embodiment of the present disclosure have been described above. Hereinafter, the present disclosure will be described more specifically through experimental examples.

### <Experimental Example 1: Preparation of metal-organic framework and porous magnetic carbon composite>

Transparent waste PET bottles with labels removed were crushed into small particles using a crusher, and particles with a size of 1.0 mm or smaller were screened using a sieve. After adding 13.017 g of Al(NO₃)₃·9H₂O and 14.0188 g of Fe(NO₃)₃·9H₂O to 50 mL of deionized water in a 100-mL high-temperature, high-pressure Teflon container, the mixture was stirred at room temperature for about 20 minutes until the two metal salts were dissolved completely. After injecting 3.3405 g of the waste PET bottle powder to the solution, the mixture was stirred at 500 rpm for about 1 hour. The molar ratio was set to 1.0 Al : 1.0 Fe : 0.5 TPA : 80 H₂O. Then, the aqueous solution wherein the two metal salts and the waste PET bottle powder were mixed was put in a stainless steel autoclave. After conducting hydrothermal synthesis in an oven at 220 °C for 3 days, the reaction product was allowed to cool naturally to room temperature. The final product was centrifuged, washed several times using deionized water and ethanol, and then dried in vacuo at 150 °C for 12 hours.

After conducting heat treatment at 330 °C for 3 days (heating rate = 5 °C/min) under general air condition for activation, an aluminum-based metal-organic framework with iron oxide bonded (named 'α-Fe₂O₃/MIL-53(Al)') was prepared by cooling naturally to room temperature.

A porous magnetic carbon composite with alumina and magnetite bonded (named 'Al-Fe based MOs@C') was synthesized by heat-treating the α-Fe₂O₃/MIL-53(Al) at 600 °C, 700 °C or 800 °C for 4 hours (heating rate = 5 °C/min) under nitrogen atmosphere (nitrogen gas injection rate = 25 mL/min).

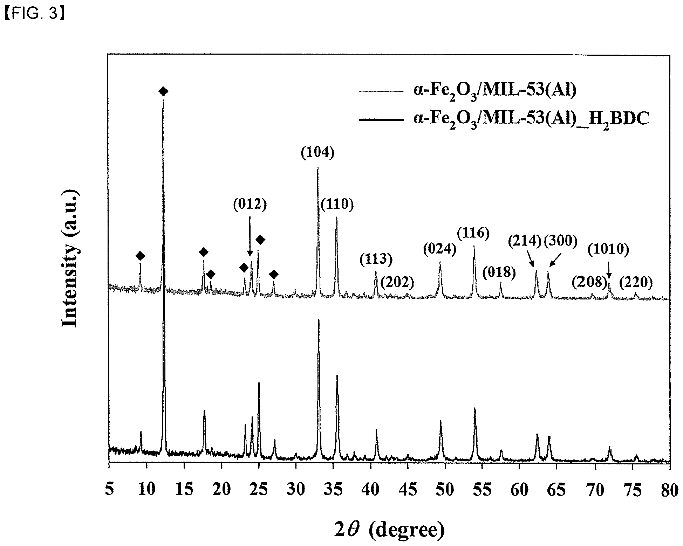

From XRD analysis of the aluminum-based metal-organic framework with iron oxide bonded synthesized in Experimental Example 1 (α-Fe₂O₃/MIL-53(Al)), it was confirmed that the XRD analysis result was exactly the same as that of an aluminum-based metal-organic framework (α-Fe₂O₃/MIL-53(Al)_H₂BDC) prepared using commercially available terephthalic acid (TPA) (see FIG. 3). In addition, it was confirmed that the aluminum-based metal-organic framework with α-Fe₂O₃ bonded was synthesized successfully because the peak of hematite (α-Fe₂O₃) was observed in the XRD analysis result shown in FIG. 3.

### <Experimental Example 2: Change in physical properties depending on activation temperature>

In order to investigate the change of the metal-organic framework depending on activation temperature, the metal-organic framework was activated at 330 °C, 350 °C or 400 °C, and then XRD analysis and thermogravimetric analysis were conducted.

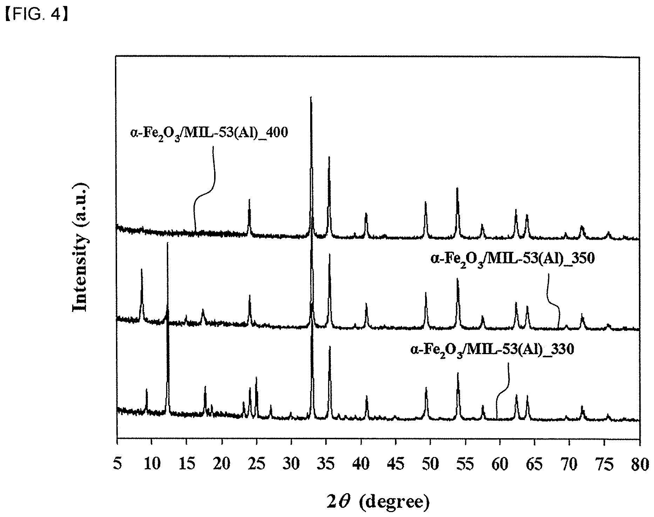

Referring to the XRD analysis result of FIG. 4, the major peak of MIL-53(Al) was decreased gradually as the activation temperature was increased. When the activation temperature was 400 °C, all peaks disappeared except the peaks related with α-Fe₂O₃.

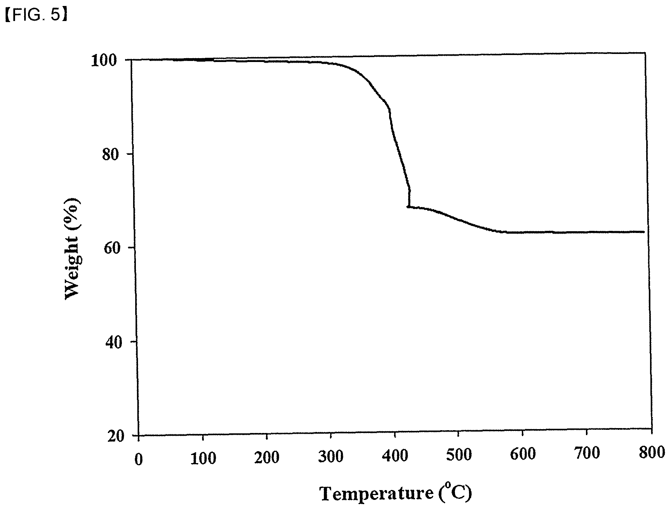

The XRD analysis result is closely related with the thermogravimetric analysis result. Referring to the thermogravimetric analysis result of FIG. 5, about 35% of weight loss occurred between 350 °C and 400 °C, because the terephthalic acid constituting the metal-organic framework was thermally decomposed. Accordingly, the activation temperature needs to be 300-350 °C in order to ensure the structural stability of the metal-organic framework through activation.

### <Experimental Example 3: Analysis of characteristics of porous magnetic carbon composite depending on sintering temperature>

XRD analysis was conducted after preparing a porous magnetic carbon composite with alumina and magnetite bonded by sintering the α-Fe2O3/MIL-53(Al) prepared in Experimental Example 1 at 600 °C, 700 °C or 800 °C for 4 hours under nitrogen atmosphere.

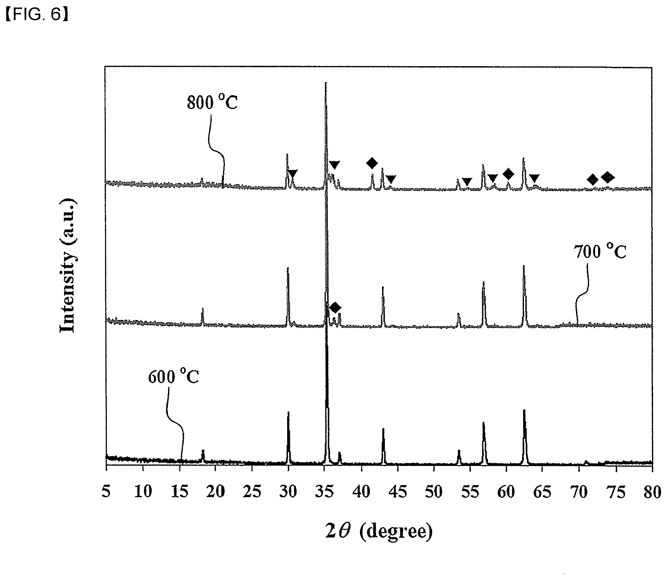

Referring to the XRD analysis result of FIG. 6, it was confirmed that only Fe₃O₄ was present when the sintering was performed at 600 °C. No aluminum oxide (e.g., Al₂O₃)-related peak was observed, which suggests that aluminum oxide is present in amorphous state. In addition, it was observed that FeO ( ◆) was formed as the sintering temperature was increased and FeAl₂O₄ (▼) was formed at 800 °C. It is because α-Fe₂O₃ was converted (α-Fe₂O₃ → Fe₃O₄ → FeO) as the sintering temperature was increased due to reduction at high temperature. At the highest sintering temperature of 800 °C, some of Al₂O₃ reacted with FeO existing in the porous magnetic carbon composite to form FeAl₂O₄.

### <Experimental Example 4: Assessment of adsorption performance>

The adsorption performance of the porous magnetic carbon composite prepared in Experimental Example 1 (Al-Fe based MOs@C) for four NSAIDs, i.e., ibuprofen (IBU), diclofenac (DIC), naproxen (NAP) and ketoprofen (KET), and one heavy metal (Cr(Vl)) was assessed.

The initial concentration was set to 10 ppm, the Al-Fe based MOs@C injection rate to 0.02 g/40 mL, reaction temperature to 20 °C, and reaction time to 24 hours.

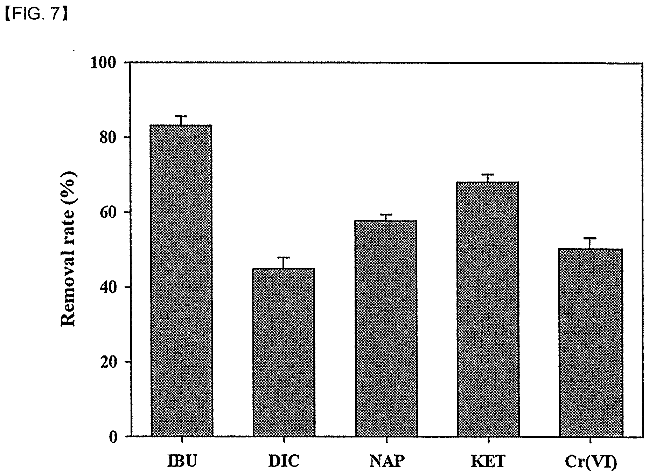

As shown in FIG. 7, the removal rate was IBU for 83.3%, 44.8% for DIC, 57.8% for NAP, and 68.1% for KET. The removal rate of Cr(VI) was 50.4%. Through this result, it can be seen that the porous magnetic carbon composite according to the present disclosure is effective for removing organic pollutants and heavy metals in water.

It is thought that adsorption of NSAIDs to the porous magnetic carbon composite is achieved through electrostatic attraction, π-π bonding, hydrogen bonding, etc. in addition to adsorption into the pores of the porous magnetic carbon composite. In addition, it is thought that Cr(VI) is adsorbed by pore adsorption, electrostatic attraction, anion exchange or inner sphere surface complexation.

### <Experimental Example 5: Assessment of porosity>

Nitrogen adsorption/desorption experiment was performed to assess the porosity of the porous magnetic carbon composites (Al-Fe based MOs@C) prepared at different sintering temperatures in Experimental Example 1.

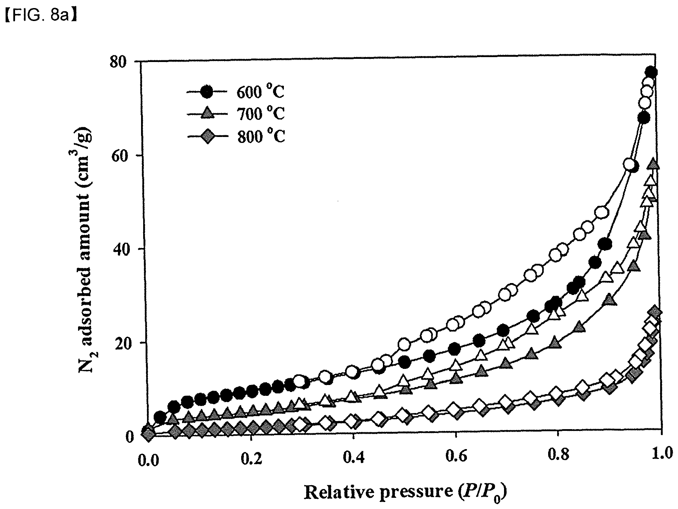

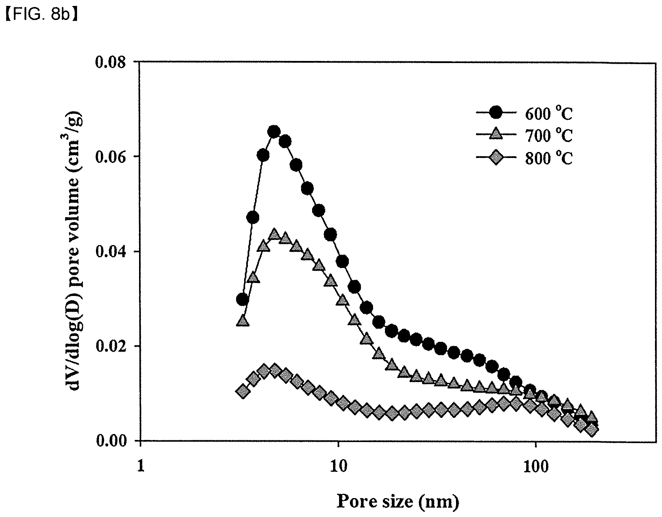

Referring to FIG. 8A, FIG. 8B and Table 1, it was confirmed that porosity was decreased as the sintering temperature was increased. It is thought the reason is because the pore was clogged by nanosized aluminum, which melts at about 660 °C or higher, as the sintering temperature was increased.

**[Table 1]**

| <Pore characteristics of porous magnetic carbon composite depending on sintering temperature> | | | |
|---|---|---|---|
| Sintering temperature (°C) | Specific surface area (m²/g) | Pore volume (cm³/g) | Average pore diameter (nm) |
| 600 | 34.21 | 0.116 | 9.85 |
| 700 | 18.90 | 0.083 | 10.76 |
| 800 | 5.88 | 0.038 | 13.94 |

### <Experimental Example 6: Assessment of magnetism recovery of porous magnetic carbon composite>

Magnetism recovery test was conducted for the porous magnetic carbon composite (Al-Fe based MOs@C) prepared at a sintering temperature of 600 °C in Experimental Example 1 using a vibrating-sample magnetometer (VSM). The test was conducted at room temperature by applying a magnetic field of -30 to 30 K Oe.

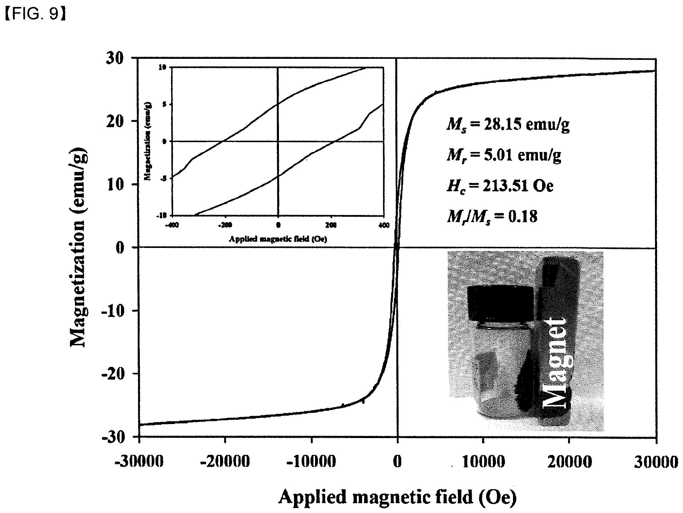

As a result, saturation magnetization (Ms) was 28.15 emu/g and the Mr/Ms ratio was 0.18 as shown in FIG. 9, suggesting that the composite has superparamagnetic characteristics. As shown in the insert of FIG. 9, the composite could be easily separated using a magnet.

## Claims

1. A method for preparing an aluminum-based metal-organic framework with iron oxide bonded using waste PET bottles, comprising:
a step of preparing an aqueous solution wherein a powder of waste PET bottles, an aluminum salt and an iron salt are mixed; and
a step of forming an aluminum-based metal-organic framework with an iron oxide (α-Fe₂O₃) bonded by conducting hydrothermal synthesis at a temperature satisfying both the depolymerization temperature of PET and the synthesis temperature of an aluminum-based metal-organic framework in the aqueous solution,
wherein, in the step wherein the aluminum-based metal-organic framework with an iron oxide (α-Fe₂O₃) bonded is formed by hydrothermal synthesis,
the depolymerization temperature range of PET overlaps with the temperature range where the aluminum-based metal-organic framework is synthesized, and the hydrothermal synthesis is conducted within the overlapping temperature range, and
the aluminum-based metal-organic framework is formed by reaction between terephthalic acid and aluminum as terephthalic acid is formed from the waste PET bottles, and an iron component of the iron salt is converted to α-Fe₂O₃ (hematite) and then bonded with the aluminum-based metal-organic framework.

2. The method for preparing an aluminum-based metal-organic framework with iron oxide bonded using waste PET bottles according to claim 1, wherein the temperature range where the depolymerization temperature range of PET overlaps with the temperature range where the aluminum-based metal-organic framework is synthesized is 200-240 °C.

3. The method for preparing an aluminum-based metal-organic framework with iron oxide bonded using waste PET bottles according to claim 1 or 2, wherein the aluminum salt and the iron salt comprise a nitrate group (NO₃⁻), the aluminum salt is Al(NO₃)₃·9H₂O and the iron salt is Fe(NO₃)₃·9H₂O.

4. The method for preparing an aluminum-based metal-organic framework with iron oxide bonded using waste PET bottles according to any of claims 1 to 3, which comprises a step of, in the state where the aluminum-based metal-organic framework has been formed by hydrothermal synthesis, removing unreacted terephthalic acid remaining in the aluminum-based metal-organic framework and inducing activation of the aluminum-based metal-organic framework at the same time by activating the aluminum-based metal-organic framework at a predetermined temperature, wherein the activation temperature of the aluminum-based metal-organic framework is 300-350 °C.

5. The method for preparing an aluminum-based metal-organic framework with iron oxide bonded using waste PET bottles according to any of claims 1 to 4, wherein, in the aqueous solution wherein the powder of waste PET bottles, the aluminum salt and the iron salt are mixed, they are mixed at a molar ratio Al: Fe : TPA (terephthalic acid) : H₂O = 1 : 1 : 0.3-0.7 : 48-112, and the addition amount of the powder of waste PET bottles is determined based on the molar ratio.

6. A method for preparing a porous magnetic carbon composite, comprising:
a step of preparing an aqueous solution wherein a powder of waste PET bottles, an aluminum salt and an iron salt are mixed;
a step of forming an aluminum-based metal-organic framework with an iron oxide (α-Fe₂O₃) bonded by conducting hydrothermal synthesis at a temperature satisfying both the depolymerization temperature of PET and the synthesis temperature of an aluminum-based metal-organic framework in the aqueous solution; and
a step of forming a porous magnetic carbon composite with alumina and magnetite bonded by sintering the aluminum-based metal-organic framework with iron oxide bonded,
wherein, in the step wherein the aluminum-based metal-organic framework with an iron oxide (α-Fe₂O₃) bonded is formed by hydrothermal synthesis,
the depolymerization temperature range of PET overlaps with the temperature range where the aluminum-based metal-organic framework is synthesized, and the hydrothermal synthesis is conducted within the overlapping temperature range.

7. The method for preparing a porous magnetic carbon composite according to claim 6, wherein, in the step wherein the aluminum-based metal-organic framework with an iron oxide (α-Fe₂O₃) bonded is formed by hydrothermal synthesis, the aluminum-based metal-organic framework is formed by reaction between terephthalic acid and aluminum as terephthalic acid is formed from the waste PET bottles, and an iron component of the iron salt is converted to α-Fe₂O₃ (hematite) and then bonded with the aluminum-based metal-organic framework.

8. The method for preparing a porous magnetic carbon composite according to claim 6 or 7, wherein, as a result of the sintering of the aluminum-based metal-organic framework with iron oxide bonded, an organic ligand compound is converted to a carbon-based material, an aluminum compound of the metal-organic framework is converted to alumina (Al₂O₃) and the iron oxide (α-Fe₂O₃) bonded to the metal-organic framework is converted to magnetite (Fe₃O₄) with the porous structure of the metal-organic framework maintained.

9. The method for preparing a porous magnetic carbon composite according to any of claims 6 to 8, wherein the sintering temperature of the aluminum-based metal-organic framework with iron oxide bonded is 600-800 °C.

10. The method for preparing a porous magnetic carbon composite according to any of claims 6 to 9, wherein the temperature range where the depolymerization temperature range of PET overlaps with the temperature range where the aluminum-based metal-organic framework is synthesized is 200-240 °C.

11. The method for preparing a porous magnetic carbon composite according to any of claims 6 to 10, wherein the aluminum salt and the iron salt comprise a nitrate group (NO₃⁻), the aluminum salt is Al(NO₃)₃·9H₂O and the iron salt is Fe(NO₃)₃·9H₂O.

12. The method for preparing a porous magnetic carbon composite according to any of claims 6 to 11, which comprises a step of, in the state where the aluminum-based metal-organic framework has been formed by hydrothermal synthesis, removing unreacted terephthalic acid remaining in the aluminum-based metal-organic framework and inducing activation of the aluminum-based metal-organic framework at the same time by activating the aluminum-based metal-organic framework at a predetermined temperature, wherein the activation temperature of the aluminum-based metal-organic framework is 300-350 °C.

13. The method for preparing a porous magnetic carbon composite according to any of claims 6 to 12, wherein, in the aqueous solution wherein the powder of waste PET bottles, the aluminum salt and the iron salt are mixed, they are mixed at a molar ratio Al: Fe: TPA (terephthalic acid) : H₂O = 1 : 1 : 0.3-0.7 : 48-112, and the addition amount of the powder of waste PET bottles is determined based on the molar ratio.

14. A porous magnetic carbon composite with alumina and magnetite bonded formed by sintering of an aluminum-based metal-organic framework with iron oxide bonded, wherein the aluminum-based metal-organic framework with iron oxide bonded is formed by hydrothermal synthesis of an aqueous solution wherein a powder of waste PET bottles, an aluminum salt and an iron salt are mixed.
